# EUROPEAN PATENT APPLICATION

(11) **EP 2 636 752 A1**
(43) Date of publication of application: **11.09.2013**
(21) Application number: 12158331.4
(22) Date of filing: 06.03.2012
(51) Int. Cl.: C12Q 1/68, G01N 33/574

(54) **In vitro method for determining disease outcome in pulmonary carcinoids.**

(71) Applicant: Universiteit Maastricht, 6211 LK Maastricht (NL); Academisch Ziekenhuis Maastricht, 6229 HX Maastricht (NL)
(72) Inventor: The designation of the inventor has not yet been filed
(74) Representative: Habets, Winand

(57) **Abstract**

The invention is in the field of molecular diagnostics and provides markers for predicting disease outcome in pulmonary carcinoids. More in particular, the invention provides an in vitro method for predicting the outcome of a disease by determining the level of a marker in a sample wherein the disease is a pulmonary carcinoid tumor and wherein the marker is an RNA or protein expression product of a RET proto-oncogene or an RNA or protein expression product of an Orthopedia homeobox (OTP) gene

## Description

### Field of the invention

The invention is in the field of molecular diagnostics and provides markers for predicting disease outcome in pulmonary carcinoids.

### Background of the invention

Pulmonary carcinoids comprise a group of well-differentiated neuroendocrine tumors (NETs) with little relation to cigarette smoking.(1) In contrast to other lung NETs, i.e. the poorly differentiated large cell neuroendocrine carcinoma (LCNEC) and small cell lung cancer (SCLC), carcinoids are characterized by a low metastatic rate and a relatively favorable prognosis. According to the WHO, based on histopathological features, lung carcinoids are subclassified as typical carcinoids (TCs) or atypical carcinoids (ACs) (2) ACs are more often characterized by malignant behavior and have a lower 5-year survival rate compared to TCs (61-88% and 92-100%, respectively).(3) However, classification of pulmonary carcinoids have been proven to be difficult, even when executed by experienced pathologists, resulting in a high interobserver variability.

Alternative ways to sub-classify carcinoids into categories relevant for prognosis is therefore desired. Metastases will develop in 4-64% of carcinoid patients (TCs: 4-14%, ACs: 35-64%), usually in regional lymph nodes, but also at distant sites including liver, bones, brain, subcutaneous tissue and breast.(3,4). Although most patients remain cancer-free within five years after surgery, there is a risk on late recurrence and/or distant metastasis, the occurrence of which cannot be sufficiently predicted by the current WHO classification. The use of chemotherapy or radiation therapy for patients with metastatic disease has limited curative potential.

A few studies have reported clinical and molecular parameters associated with higher risks for developing metastases or a poor disease outcome. Clinical parameters with prognostic value include size ≥ 3.5 cm, co-secretion of peptides, degree of differentiation, mitotic index, metastases and presence of necrosis (5,6). Furthermore, higher protein expression of the proliferation marker Ki67 and the anti-apoptosis protein Bcl-2 has been shown to be discriminative between recurrent and nonrecurrent carcinoid tumors (7). An additional study on 121 pulmonary NETs including 21 carcinoids showed a shift to low Bax and high Bcl-2 expression levels in association with ACs, resulting in an unfavorable prognosis (8). Also, it has been reported that a decreased protein expression of the standard variant of the adhesion molecule CD44 (CD44s) is associated with a decreased survival for TC patients (9). This latter study used older criteria for classification and is thereby likely to have included also ACs. Dislocation of two other adhesion molecules from the membrane, i.e. E-cadherin and β-catenin, is reported to be associated with lymph node metastasis in ACs (10).

In previous studies, (array) comparative genomic hybridization (CGH) has been performed to determine genomic abnormalities in lung carcinoids in general, and to make a comparison between TCs and ACs. Genomic alterations contribute to carcinogenesis by changing the expression levels of critical oncogenes and tumor suppressor genes. In lung carcinoid tumor biology, mainly multiple endocrine neoplasia type 1 (MEN1) gene mutations and/or chromosome 11q deletions have been demonstrated (11-17).

MEN1 is a syndrome in which an inherited mutation within the *MEN1* gene, located at 11q13, predisposes to the formation of multiple NETs. Because in - 5% of MEN1 patients formation of bronchial carcinoids has been observed (18), functional inactivation of Menin, the *MEN1* gene product, has been implicated in the tumorigenesis of sporadic lung carcinoids. The somatic *MEN1* mutation rate is approximately 18%, but frequencies of loss of heterozygosity (LOH) at 11q13 and more telomeric regions are higher, pointing to the presence of other tumor suppressor genes at this chromosome arm, both at 11q13 and at the telomeric part of 11q.(12,19).

There is still a need in the art for better markers suitable for the prediction of disease outcome in pulmonary carcinoids.

### Summary of the invention

In order to identify novel prognostic markers for pulmonary carcinoids, we performed high resolution expression profiling on five tumors from patients with a very poor disease outcome in comparison with five carcinoids from patients with a favorable outcome, independent of histopathologic subclassification into TCs and ACs. We were able to identify two genes whose expression levels differed very strongly between these two groups, including the *OTP* gene, which was downregulated, and the upregulated *RET* proto-oncogene.

We validated the prognostic value of these markers by performing RT-PCR on a series of frozen cases and by immunohistochemistry on a very large series of formalin-fixed paraffin-embedded (FFPE) tissue sections and tissue microarrays of both carcinoid tumors and high grade NE carcinomas in comparison, and showed strong associations with disease outcome.

Hence, the invention relates to an in vitro method for predicting the outcome of a disease by determining the level of a marker in a sample wherein the disease is a pulmonary carcinoid tumor and wherein the marker is an RNA or protein expression product of a RET proto-oncogene or an RNA or protein expression product of an Orthopedia homeobox (OTP) gene.

### Detailed description of the invention

Few prognostic markers have so far been identified for pulmonary carcinoid tumors (23,24). Here we report two novel, strong prognostic candidates for these tumors, i.e. the Orthopedia homeobox (*OTP*) and the *RET* proto-oncogene. The novel markers were strongly associated with an adverse patient outcome. The markers OTP and RET outperformed the previously identified marker CD44.

The *OTP* gene has been previously described. In summary: Orthopedia (OTP) is an evolutionary conserved homeodomain-containing transcription factor (33) for which currently functions have been described only within the central nervous system. OTP has important roles in the development of the neurosecretory system in the hypothalamus(34). In the mouse, it is involved in the terminal differentiation of neuroblasts (35) The zebrafish ortholog of OTP is regulated by the zinc-finger transcription factors Fezl and Pac1,(36) while downstream targets of murine Otp include possibly Brn2 and Sim2, both required for the development of the hypothalamic-pituitary axis (34,35).

Otp-/- mutant mice died within three days after birth with clear abnormalities of the hypothalamus and a reduction in brain volume (35) Especially differentiation, proliferation and migration of Otp-expressing cells was severely abrogated in these mice (34) Recently, Amir-Zilberstein et al. (37) reported that zebrafish and mouse Otp can be induced by stress, leading to recruitment of Otp together with pCREB to the promoters of corticotropin-releasing hormone and the splicing factor ataxin 2-binding protein-1 (A2BP1). Interestingly, A2BP1 regulates the alternative splicing of pac1, which was previously hypothesized to regulate also Otp posttranscriptionally (36,37).

The short splice variant of PAC1 regulates moreover the transcriptional activation of CRH, while the longer variant terminates the stress-induced CRH transcription (37).

We show here the presence of both the human *OTP* gene and protein OTP in lung carcinoid tumors with a favorable disease outcome. Nuclear OTP expression may be regarded as a hallmark of the NE nature of these carcinoid tumors. Normal lung tissue, epithelial cell layers, ganglions and macrophages showed cytoplasmic positivity. Furthermore, additional normal and malignant tissues stained in the human protein atlas (http://www.proteinatlas.org/) only displayed cytoplasmic positivity.

On the other hand, gene expression was almost absent in NE cell lines and most high grade NE carcinomas. Also a subset of malignant carcinoids was clearly negative. With respect to protein expression of OTP, especially the nuclear expression was directly coupled to the mRNA expression (p<0.0001).

Our data show a gradual loss of OTP expression within lung NETs, where cases with relatively benign tumors have strong nuclear and cytoplasmic OTP expression, tumors with potentially dangerous properties have lost nuclear OTP expression and very malignant tumors loose both nuclear and cytoplasmic staining. Furthermore, diffuse idiopathic pulmonary neuroendocrine cell hyperplasia (DIPNECH), a presumed carcinoid precursor lesion, showed strong nuclear positivity. With respect to other NETs, insulinomas were negative, as well as an adrenocortical adenoma and carcinoma.

The *RET gene* also has been previously described. In summary: The *RET* (rearranged during transfection) proto-oncogene is located at chromosome 10q11.2 and encodes a receptor tyrosine kinase with many downstream targets (38,39). Both gain and loss of function of the RET protein is implicated in disease, and leads to multiple endocrine neoplasia type 2 (MEN2) and Hirschprung disease, respectively (38). In contrast to the loss of function mutations of the tumor suppressor gene *MEN1,* which cause the MEN1-syndrome as discussed above, and are randomly spread over the *MEN1* gene, (40) *RET* mutations involved in the MEN2-syndrome are gain of function mutations mainly located at two hotspots in the gene. Depending on the genetic location of the *RET* mutation, three subtypes of the MEN2-syndrome can be distinguished, i.e. MEN2A, MEN2B and familial isolated medullary thyroid cancer (FMTC).

In the MEN2A syndrome, 90% of the mutations occur in codons encoding six extracellular cysteines,(38,39) while the MEN2B syndrome affects almost always one and the same cytoplasmic amino acid, i.e. Met918Thr. In both the MEN2A and MEN2B syndrome, the most frequently occurring neoplasm is medullary thyroid carcinoma (MTC),(38,41) but cases associated with MEN2B display a much more aggressive course, possibly caused by strong activation of Jun NH2-terminal protein kinase (JNK) by the activated RET via Dok1, which has a higher affinity for MEN2B mutated *RET* (41).

Pheochromocytomas can occur in association with both MEN2 subsyndromes, while hyperplasia of the parathyroid arises only in association with MEN2A. The third subtype, FMTC, is characterized by only MTC tumors that occur later in life.(39,41).

Next to the involvement of *RET* in these familial tumors, *RET* mutations have also been reported in sporadic cancers. They occur in 40-50% of sporadic MTCs and in a small fraction of pheochromocytomas (38,42). Few studies have assessed *RET* mutation in other NETs. Komminoth et al. (42) did not find mutations in 11 lung carcinoid tumors and 7 SCLCs. In publications by Futami et al.(43,44), two identical mutations (Ala to Asp at codon 664) were identified in two out of six SCLC cell lines and their respective primary tumors, but these authors failed to identify further mutations in an additional 12 SCLC patients (45). Another study also did not identify *RET* mutations in 54 SCLC cell lines, although they identified *RET* expression in 57% of these cell lines, in contrast to our findings in which we did not observe upregulation of *RET* in SCLC tumor samples and NE cell lines (46). With the exception of the study by Komminoth et al.(42), pulmonary carcinoids were not analyzed for *RET* mutations.

We analyzed the mutational hotspots (exon 10, 11 and 16) within the *RET* gene of the ten carcinoid cases that were used for the cDNA microarrays, but did not identify any mutation, neither in the cases with low *RET* expression, nor in the cases with high expression.

Menin and RET have some opposing functions with respect to activation of the Wnt-signaling inducing protein β-catenin. While the Menin protein directly binds β-catenin and transports it out of the nucleus via nuclear-cytoplasmic shuttling to suppress Wnt-signalling, which is stimulated by nuclear β-catenin (47),RET binds to and phosphorylates also directly beta-catenin (48). Via this RET-specific phosphorylation, β-catenin is able to accumulate in the nucleus and activate the expression of e.g. cyclin D1, EGFR and JunB, typical RET targets. While RET promotes a nuclear localization of beta-catenin, Menin removes it from the nucleus and targets it to the membrane (47).

### Legend to the figures

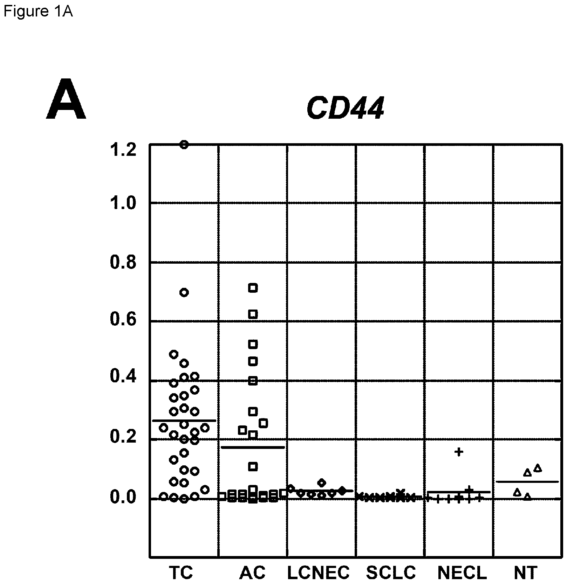

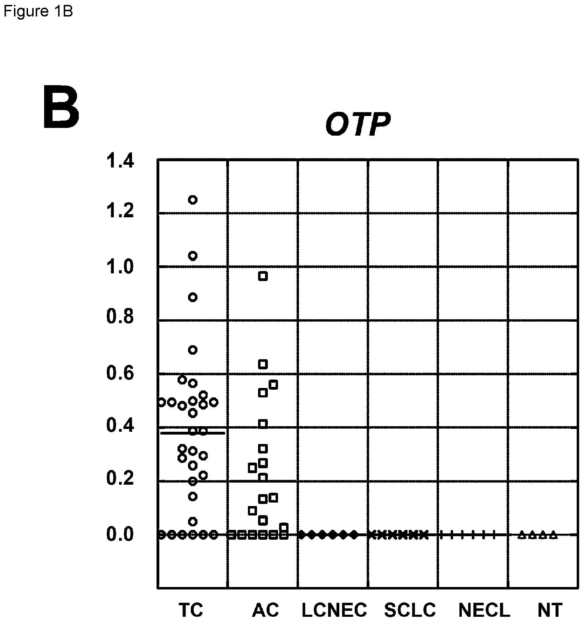

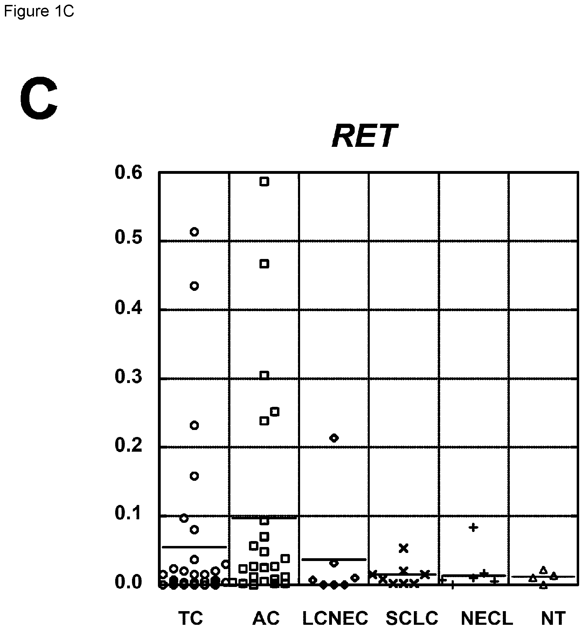

### Figure 1 - RT-PCR results

Scatterplots showing the relative mRNA expression values (Y-axis) of *CD44* (A), *OTP* (B) and *RET* (C) compared to the geometric mean of the expression levels of the housekeeping genes *ACTB* and *CYPA;* for typical carcinoids (TC, circles), atypical carcinoids (AC, squares), large cell neuroendocrine carcinomas (LCNEC, diamonds), small cell lung cancers (SCLC, crosses), neuroendocrine cell lines (NECL, plus signs) and normal tissues (NT, triangles). The mean relative expression levels for the respective subgroups are indicated by straight lines.

### Figure 2 - Survival analyses for Pulmonary carcinoids using RT-PCR data

Kaplan-Meier analyses depicting the difference in survival for high (straight lines) or low (dotted lines) mRNA expression of *CD44* (cut-off value 0.0227, A), *OTP* (cut-off value 0.111, B), and *RET* (cut-off value 0.0221, C).

### Figure 3 - Immunohistochemistry results.

Panel A shows a carcinoid case that was included in the microarray from which the patient had a favorable disease outcome. This case shows a very strong membranous staining in all cells and was typical for carcinoid tumors with a favorable disease outcome. None of the high grade lung NETs showed this staining pattern, with the exception of the proximity of necrotic areas.

Panel B shows a carcinoid case that was included in the microarray from which the patient had an adverse disease outcome. This case has complete absence of membranous CD44 staining. However, this case does have a weak nuclear staining in a subset of cells (5-10%).. This nuclear staining for CD44 was rare (7% of membrane-negative cases), but absence of membranous staining was a frequent event and the large majority of high grade lung NETs had no membranous CD44 staining. (C-E): OTP.

Panel C shows a carcinoid case from which the patient had a favorable disease outcome. This case shows both strong nuclear and cytoplasmic positivity, in agreement with the RT-PCR results. When nuclear staining was prominent cytoplasmic staining could be either strong as in the depicted case, but could be also weakly present or almost absent.

Panel D shows a carcinoid case from which the patient is alive with no evidence of disease. In this case, nuclear OTP expression is gradually lost. On the left on the picture the OTP expression is prominently present in the majority of nuclei, while on the left nuclear OTP expression is completely lost. Cytoplasmic OTP staining is present in the complete section.

Panel E shows an atypical carcinoid case from which the patient is currently alive is shown. This case has lost nuclear immunhistochemistry in most of the cells. It retains cytoplasmic positivity however in the majority of cells.

Panel F shows a typical carcinoid from which the patient is deceased. This case has lost OTP expression completely, both nuclear and cytoplasmic. The majority of malignant carcinoid tumors and almost all high grade neuroendocrine carcinomas were completely OTP negative.

Panel G shows one of the cases with a poor prognosis that was included in the microarray analysis. This sample, as well as most of the other samples tested, has a punctuate cytoplasmic RET staining pattern. None of the cases tested displayed membrane-localized RET.

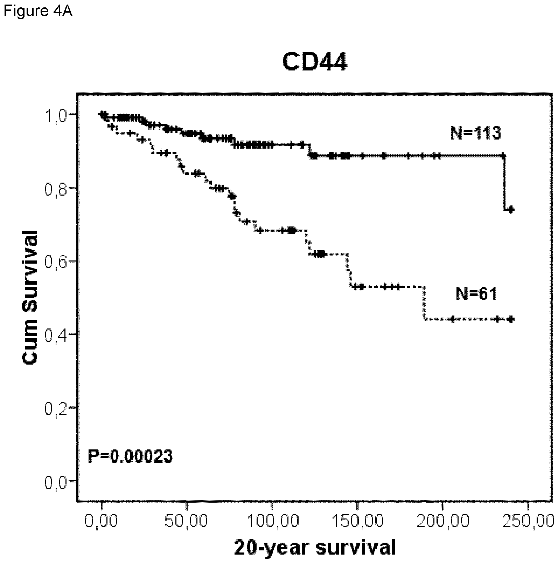

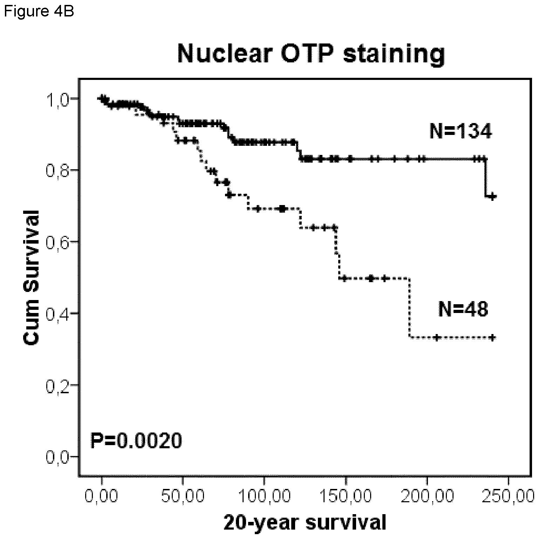

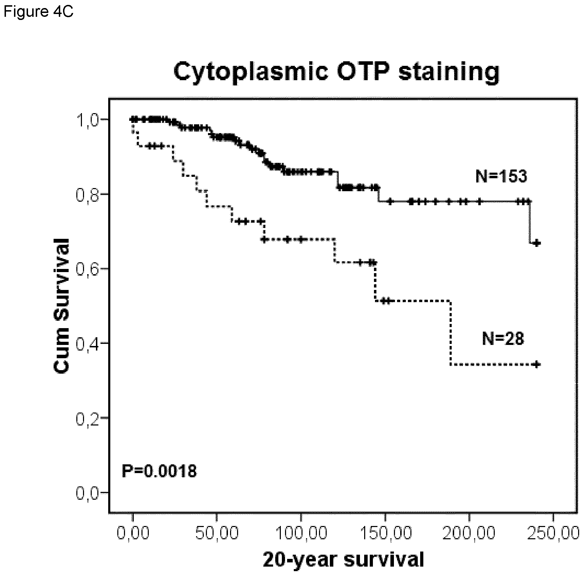

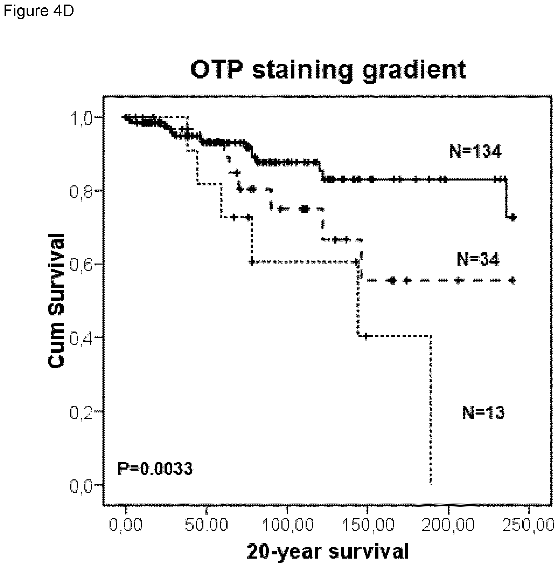

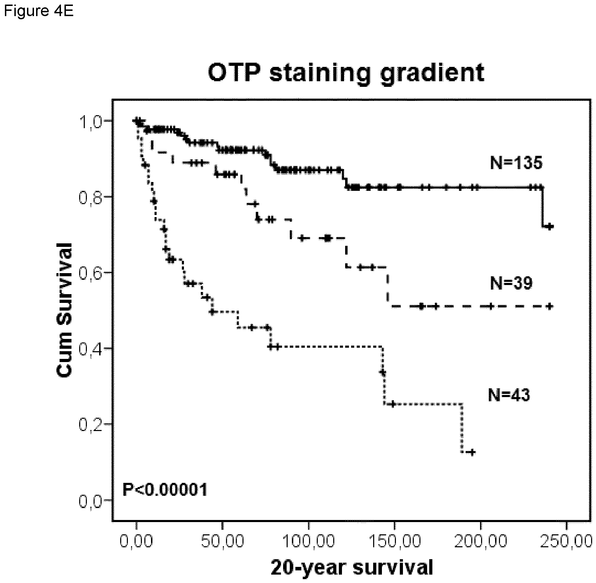

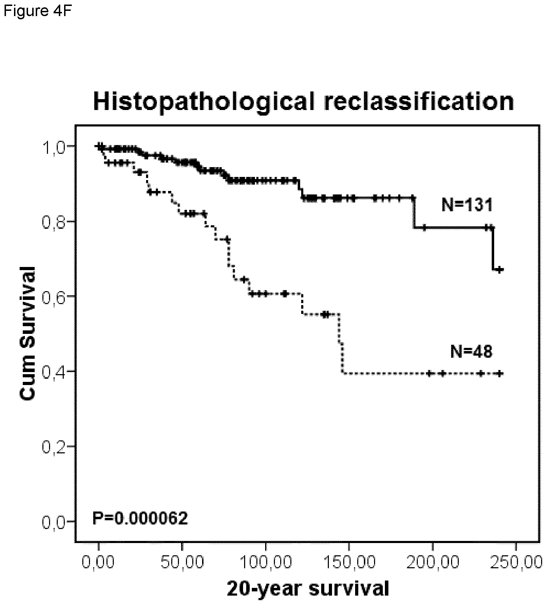

### Figure 4 - Survival analyses for pulmonary carcinoids using immunohistochemistry data.

Kaplan-Meier analyses showing 20-year overall survival for carcinoid tumors (A-D) or a combined group of carcinoid tumors and high grade neuroendocrine carcinomas (E). A) CD44 staining. Dotted line indicate staining intensities of 1 and lower, while the solid line depict cases with staining intensities of more than 1. B) Nuclear OTP staining. Dotted line indicate staining intensities of 1 and lower, while the solid line depict cases with staining intensities of more than 1. C) Cytoplasmic OTP staining. Dotted line indicate staining intensities of 1 and lower, while the solid line depict cases with staining intensities of more than 1. D-E) Gradient of OTP staining for carcinoids (D) and a combined group of carcinoids and high grade lung neuroendocrine tumors (E). The solid line indicates cases with nuclear staining, irrespectively of cytoplasmic staining (which was most often present), the dashed line indicates tumors which have lost nuclear staining but retain cytoplasmic staining, and the dotted line indicates tumors that have lost both nuclear and cytoplasmic staining. F) Histopathology after reclassification. Solid line indicates TCs and dotted line comprises ACs.

### Examples

### Experimental procedures

### Collection of tumor material and clinical data

We collected frozen material from 31 TCs, 2 unclassified carcinoid tumors, 23 ACs, 7 LCNECs, 1 mixed LCNEC-SCLC and 8 SCLCs. With respect to the carcinoid cases, 19 males and 35 females (2 unknown) were included with a mean age of 53 years (range 21-83). Regarding the cases of high grade lung NET, 12 males and 1 female (3 unknown) were included with a mean age of 67 years (range 48-79). All material consisted of at least 70% tumor cells.

From these series we selected five tumors from patients with a favorable prognosis (all ≥ 7 year disease-free survival without metastasis or recurrence) and five from carcinoid patients with a very poor disease outcome (all death from disease). We decided to analyze the latter cases independently from their histopathological classification. All carcinoids with a poor prognosis were ACs, but within the group of five carcinoids with a favorable disease outcome, there were also two ACs included with an excellent disease outcome. Material from these cases was subjected to gene expression profiling using Agilent Human* GE 4x44K v2 Microarrays (Agilent Technologies, Santa Clara, CA, USA).

In addition, we analyzed FFPE tissue from the above mentioned series of carcinoid tumors with frozen tissue available, for which both frozen and paraffin tissue was available. We collected in addition FFPE tissue from a second large series of pulmonary carcinoid tumors and high grade lung NETs, including a number of tissue micro arrays and tumor sections, in total comprising 147 TCs, 50 ACs, 20 LCNECs and 24 SCLCs. With respect to the carcinoids, there were 90 males and 110 females and the mean age was 52 years (range 16-83). For the high grade lung NETs, there were 24 males and 7 females and the mean age was 64 years (range 41-79). All patient material was used according to the Code for Proper Secondary Use of Human Tissue (Federation of Medical Scientific Societies, The Netherlands; 2003) and was classified according to histopathological features (TC, no necrosis and a mitotic index < 2 per 2 mm²; AC, necrosis or a mitotic index 2-10 per 2 mm²; high grade lung NETs, mitotic index >10 per 2 mm²) defined by the WHO (2).

Clinicopathological data from the majority of patients was also collected, including information concerning tumor recurrence and metastasis, overall patient survival time, and when possible information regarding cancer specific deaths.

### RT-PCR

We isolated RNA from the frozen cases using the RNeasy Mini Kit (Qiagen GmbH, Hilden, Germany). In addition, controls comprised material from 9 neuroendocrine (NE) cell lines (Bon-1, CM, H69, H295, H460, H720, H727, QGP and SW13) and MVP™ total RNA from normal human adrenal gland, liver, lung and pancreas (Stratagene, Agilent Technologies, Santa Clara, CA, USA). The Bon-1 cell-line was obtained as described previously.20 CM was kindly provided by P. Pozzilli, Rome, Italy. H295 (ATCC H295R) and SW13 were kindly provided by L. Hofland, Rotterdam, The Netherlands,21 H720 (NCl-H720) and H727 (NCl-H727) by B. Skogseid, Uppsala, Sweden, and QGP by Dieter Hoersch, Berlin, Germany. H69 and H460 were kindly provided by MUBIO technologies, Maastricht, The Netherlands.

When necessary, RNA was purified using ethanol precipitation with 0.1 × 3 M NaAc + 2.5 × 100% ethanol (-20°C). Precipitation was performed for either 30 min. at -80°C or overnight at -20°C, followed by centrifugation at 4°C for 30 min. at maximum speed. The samples were subsequently air dried by vacuum centrifugation and redissolved in nuclease-free water. RNA was conversed to cDNA by a reverse transcriptase reaction using the iScript cDNA Synthesis Kit (BioRad, Hercules, CA, USA) according to the manufacturers protocol. Quantitative real-time PCR (qRT-PCR) was performed using the following RT-PCR primers. For the housekeeping genes: ACTB, forward 5'-GCA CAG AGC CTC GCC TT-3' (SEQ ID NO: 1) and reverse 5'-GTT GTC GAC GAC GAG CG-3'; (SEQ ID NO: 2) CYPA, forward 5'-TTT CAT CTG CAC TGC CAA GAC T-3' (SEQ ID NO: 3) and reverse 5'-TTC ATG CCT TCT TTC ACT TTG C-'3; (SEQ ID NO: 4) GUSB, forward 5'-CGT CCC ACC TAG AAT CTG CT-'3 (SEQ ID NO: 5) and reverse 5'-TTG CTC ACA AAG GTC ACA GG-3'; (SEQ ID NO: 6) and HPRT, forward 5'-CAC TGG CAA AAC AAT GCA GAC T-3' (SEQ ID NO: 7) and reverse 5'-GTC TGG CTT ATA TCC AAC ACT TCG T-3' (SEQ ID NO: 8). Furthermore, with respect to the genes of interest, RT-PCR was performed using the following primer pairs: for CD44, forward 5'-GAC AAG TTT TGG TGG CAC G-3' (SEQ ID NO: 9) and reverse 5'-CAC GTG GAA TAC ACC TGC AA-3'; (SEQ ID NO: 10) for OTP, forward 5'-CGA CAT CTT TAT GCG TGA GG-3' (SEQ ID NO: 11) and reverse 5'-TTT TTG CGC TTC TTC CAC TT-3' (SEQ ID NO: 12); and for RET, forward 5'-TGC CCA GTA CCT ACT CCC TCT C-3' (SEQ ID NO: 13) and reverse 5'-GAC CAC CCC TAG CGT-3' (SEQ ID NO: 14).

All gene expressions were standardized to two housekeeping genes, which were selected to be the most constant from a total of four (ACTB, CYPA, GUSB, HPRT) that were all tested on the complete tumor series. The two most constant genes, i.e. ACTB and CYPA were chosen to be used as a reference for all other genes, after calculating a gene expression normalization factor based on the geometric mean of the expression of these two housekeeping genes with the GeNorm tool (http://medgen.ugent.be/genorm). (22) RT-PCR reactions were performed using two different commercially available SYBR green mixes (kits) depending on the characteristics of the primers. For all genes except ACTB and CYPA, the SensiMixTM SYBR & Fluorescein Kit (BioLine, London, England) was used, while the ACTB and CYPA reactions were performed using the iQTM SYBR® Green Supermix (BioRad, Hercules, CA, USA). As a positive control and to determine the optimal primer concentrations and mix (based on the primer meltcurves) a cDNA pool of the cell lines was used. ACTB, CYPA, GUSB, HPRT and RET RT-PCR reactions were performed in the Bio-Rad iQ5 cycler (BioRad) using the following program: pre-heating at 95°C for 10 min. followed by 40 cycli of 30 sec. 95°C, 30 sec. 60°C and 1 min. at 72°C (+picture), followed by 1 min. at 95°C, 1 min. at 60°C and 70 cycli of 10 sec. at 60°C increasing to 95°C (+picture) to determine the meltcurve. Data-analysis was performed using the Bio-Rad iQ5 software (version 2.0.148.60623). All other PCR reactions were performed using the Bio-Rad CFX96TM Real-Time System in combination with the C1000TM Thermal Cycler (BioRad), using the following program: pre-heating at 95°C for 10 min. followed by 40 cycli of 15 sec. 95°C and 45 sec. 60°C (+picture), followed by 1 min. at 95°C and a melting curve starting at 65°C for 5 sec. and increasing up to 95°C with 0.5°C per cycle (+picture). Data-analysis was performed using the Bio-Rad CFX Manager 2.0 software (BioRad, version 2.0.885.0923). The reproducibility of the two different RT-PCR machines was tested, and proved to be not influencing our RT-PCR results.

### Immunohistochemistry

We analyzed protein expression of CD44, a previously described marker, and OTP, on the above described second large series of FFPE tissue from pulmonary carcinoids and high grade lung NETs.

The following antibodies were used: CD44 standard variant, mouse monoclonal CD44s, clone DF1485 (DAKO, Glosturp, Denmark), in a dilution 1:50; OTP, rabbit polyclonal HPA039365 (Atlas Antibodies, Stockholm, Sweden), in a dilution 1:600; RET, rabbit polyclonal HPA008356 (Atlas Antibodies), in a dilution 1:500. All sections were deparaffinized and subsequently incubated 3 x 5 min. in water. Heat-induced epitope retrieval was performed by boiling for 20 min. in 10 mM citrate buffer (pH6). Endogenous peroxidase activity was inhibited using either 0.3% (CD44) or 3% (OTP and RET) H2O2 in methanol for 30 min.. Blocking was performed for at least 1 hour in either 3% BSA (CD44) in PBS or 5% normal goat serum in PBST (OTP and RET). Antibodies were diluted either in 1% BSA in PBS (CD44) or in 5% normal goat serum in PBST (OTP and RET). After subjection of the diluted antibody, the paraffin sections were incubated overnight at 4°C. The following day, sections were washed for 3 x 5 min. with either PBS (CD44) or PBST (OTP and RET). As a secondary antibody, Power Vision Poly-HRP goat antimouse/rabbit/rat (Immunologic, Duiven, The Netherlands) was used. The sections were incubated with the secondary antibody at 37°C for 30 min.. Afterwards, the sections were washed 2 x 5 min. with PBS and peroxidase activity was detected using 0.5 mg/ml diaminobenzidine and 2% H2O2 (EnVisionTM, DAKO) in miliQ. Sections were counterstained with hematoxylin and mounted in Entellan (Merck, Darmstadt, Germany).

All stained tumor sections were independently scored by two experienced pathologists, and in case of disagreement, a consensus was reached after analysis by a third pathologist. Staining intensities of the three proteins were divided into five categories (0-4) as follows. For the membranous staining pattern of CD44: 0 (no membrane-staining in the tumor cells), 1 (a very weak membranous staining pattern, and/or only present in single cells or small cell groups), 2 (weak to moderate staining in >50% of tumor cells), 3 (strong membranous staining around most tumor cells), 4 (very strong staining in all tumor cells). A weak nuclear staining pattern in ∼ 20% of cells (in the absence of membranous staining) was also present in a small number of cases, but did not exceed an overall staining intensity of 1. For OTP, both cytoplasmic and nuclear staining could be present and was categorized as follows: 0 (no staining), 1 (very weak overall staining [cytoplasm] or staining in single cells or very few nuclei), 2 (weak to moderate staining, for nuclear staining in >20% of nuclei), 3 (strong staining in most tumor cells), 4 (very strong staining in all tumor cells). Similar to CD44, for statistical purposes, staining intensities of both nuclear and cytoplasmic OTP of 2 or more were defined as positive. For RET, the punctuate cytoplasmic staining pattern was categorized as follows: 0 (no staining), 1 (very weak overall cytoplasmic staining and/or punctuate staining in only a few cells), 2 (weak to moderate cytoplasmic and/or punctuate staining), 3 (strong cytoplasmic and/or punctuate staining in the majority of cells), 4 (very strong cytoplasmic and punctuate staining in all cells).

### Statistical analysis

Possible correlations between clinical data and RT-PCR and immunohistochemistry results were determined using SPSS for Windows (version 15.0; SPSS Inc. 2006, Chicago, IL, USA). Cut-off points for gene expression levels were determined using area under the curve (ROC) analysis. Associations between clinical parameters (such as gender, tumor diameter, histopathologic classification and (distant) metastasis and gene or protein expression levels were analyzed using the X²-test or the Fisher's exact test, when appropriate. Mean relative gene expression levels were compared with the Student's t-test. Associations between relative gene expression levels were determined using Pearson's correlation. Survival curves were created using the Kaplan-Meier method. The log-rank test was used to test for differences between subgroups All P-values were considered statistically significant if ≤ 0.05 in two-sided tests.

### Example 1:Gene expression profiling.

Gene expression profiling identified the *RET* proto-oncogene as most strongly upregulated and the *OTP* homeobox transcription factor as most strongly downregulated in a comparison of pulmonary carcinoids with a favorable and poor disease outcome.

We examined genome-wide expression profiling data of five lung carcinoid tumors with a favorable disease outcome (defined as TxN0 tumors with at least five year disease free survival) and five lung carcinoid tumors with a very poor outcome (metastatic disease and/or deceased).

In these analyses, the orthopedia homeobox gene, *OTP,* was the most strongly downregulated gene (with a median fold change of 845). *CD44* (fold change 29), which protein expression has been reported earlier to be decreased in association with a poor prognosis in TC tumors (9), was also listed in the top-15 of downregulated genes (data not shown). The *RET* proto-oncogene was the most highly upregulated gene in lung carcinoid tumors with a poor prognosis (with a median fold change of 60).

### Example 2 Validation by RT-PCR

Validation of the prognostic value of *RET, OTP* and *CD44* for lung carcinoids was performed by RT-PCR. To prove their potential value as prognostic markers for pulmonary carcinoids and to validate the expression profiling data, *CD44* (Figure 1A), *OTP* (Figure 1 B) and *RET* (Figure 1C) gene expression levels were analyzed by RT-PCR on a series of frozen carcinoid tumor tissues, high grade lung NETs and RNA from normal tissues and NE cell lines (including the lung carcinoid cell lines H720 and H727.

In Figure 1, the RT-PCR results are provided as scatterplots. The relative expression values obtained from the cDNA microarrays for the ten carcinoid samples were perfectly reproduced by the RT-PCR data. To confirm the prognostic value of the candidate genes identified with the cDNA microarray, the RT-PCR data were correlated with clinical patient follow-up data, which was available for the majority of cases. With respect to the mean relative expression values (as compared to the levels of the housekeeping genes); these were decreased for carcinoid patients with a poor prognosis (i.e. having distant metastasis and/or being deceased) for both *CD44* (0.294 versus 0.036, respectively; p<0.0001, 95% Cl 0.166/0.349) and *OTP* (0.380 versus 0.074, respectively; p<0.0001, 95% Cl 0.176/0.437) and showed a tendency to be increased for *RET* (0.041 versus 0.151, respectively; p=0.074, 95% Cl -0.233/0.012). Relative gene expression levels of *CD44* (0.233 and 0.016, respectively; p<0.0001, 95% Cl -0.283/- 0.152) and *OTP* (0.303 and 0.000, respectively; p<0.0001, 95% Cl -0.382/-0.224) were relatively high in carcinoid tumors and almost absent in high grade NE carcinomas.

*RET* expression did not differ significantly between the two groups, but was slightly lower in high grade lung NETs as compared to carcinoids (0.028 versus 0.065, respectively). With respect to the NE cell lines, expression of CD44 was (almost) absent in most of them, except for H727 (0.029) and CM (0.157), while considerable levels of *OTP* expression were absent in all cell lines analyzed. Also *RET* expression was in general low in the cell lines, with the exception of H295 (relative expression level 0.029), H720 (0.020), H727 (0.048), and SW13 (0.029) (see also Figure 1C).

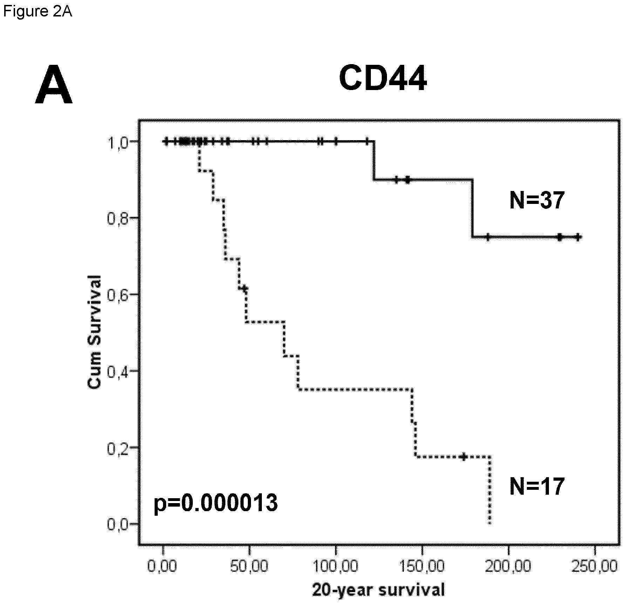

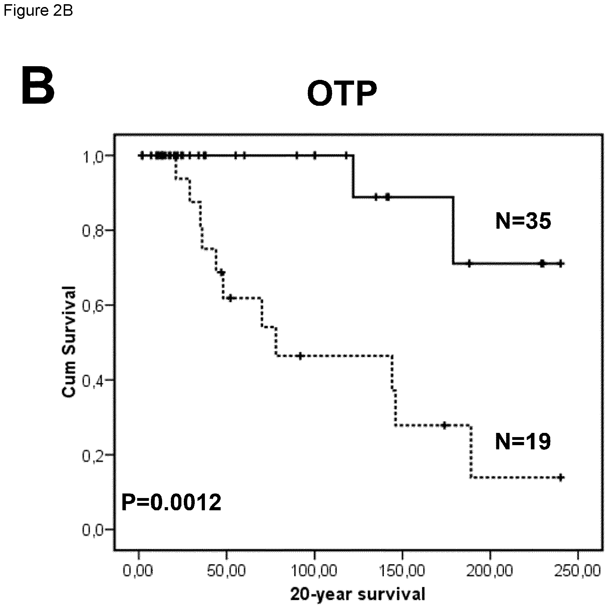

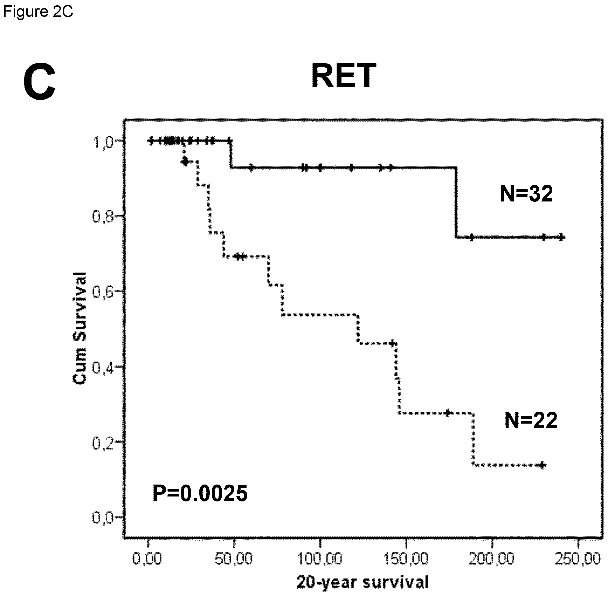

Cut-offs for survival analyses were determined using ROC area under the curve examination, and chosen to maximize sensitivity and specificity for unfavorable disease outcome. Survival analyses using these cut-off points are shown for the carcinoid tumors in Figure 2.

Upregulation of *RET* (cut-off 0.0221) and downregulation of *CD44* (cut-off 0.0227) and *OTP* (cut-off 0.111) had large impacts on 20-year overall survival, both within the complete group of NE lung tumors *(CD44,* p<0.0001; *OTP,* p<0.0001; *RET,* p=0.011) and within the carcinoids (*CD44,* p<0.0001, Figure 2A; *OTP,* p=0.0012, Figure 2B; *RET,* p=0.0025, Figure 2C). The expression values of *CD44* and *OTP* were highly correlated (Pearson's correlation, p<0.00001).

Interestingly, the *RET* oncogene was only upregulated in malignant lung carcinoid tumors, but not in high grade lung NETs, therefore resulting in a lower p-value for the group of carcinoid tumors as compared to the total group, including the carcinoids and the high grade NE carcinomas, and the above mentioned higher average *RET* exression levels in the carcinoid tumors as compared to the high grade lung NETs. For the other two genes the p-values decreased after increasing the sample size by taking into account the very aggressive lung NETs. With respect to the carcinoid tumors, patient prognosis of the frozen tumor series based on histopathologic classification into TCs and ACs was less strong than based on the novel candidate genes (p=0.029).

### Example 3; validation by immunohistochemistry.

To further validate *RET and OTP* as prognostic markers for lung carcinoids and their possible use as diagnostic markers, we performed immunohistochemistry for their encoded proteins on a second validation series consisting of both lung carcinoids and high grade NE carcinomas. This large FFPE series contained both cases that were included in the RT-PCR series (cases for which both FFPE and frozen tissue was available), and additional cases, as described in the Materials and Methods section. RET protein expression was also tested in a subset of cases. A comparison between RT-PCR and immunohistochemistry results confirmed that for *CD44, RET* and *OTP* a decrease in gene expression could be directly translated to a decrease in protein expression (Table 1). CD44 protein expression as well as nuclear OTP expression were strongly correlated to their mRNA expression (p<0.00001). Furthermore, nuclear expression of OTP was strongly correlated to CD44 protein expression (p<0.00001).

**Table 1:**

| | | **OTP** | | | **CD44** | | **RET** | |
|---|---|---|---|---|---|---|---|---|
| **Sample** | | **RT-PCR** | **Nuclear** | **Cytoplasmic** | **RT-PCR** | **IHC** | **RT-PCR** | **IHC** |
| | | **OTP** | **OTP** | **OTP** | **CD44** | **CD44** | **RET** | **RET** |
| 1 | TC | 0,0003 | 0 | 1,5 | 0,0517 | | 0,1591 | |
| 2 | TC | 0,0007 | 0 | 1 | 0,0078 | 0 | 0,5131 | |
| 3 | TC | 0,1443 | 4 | 3 | 0,1958 | 3 | 0,0004 | |
| 5 | TC | 0,4545 | 3 | 2 | 0,3666 | 3 | 0,0208 | |
| 6 | TC | 0,0011 | 0 | 2,5 | 0,0060 | 0 | 0,4344 | 2 |
| 7 | TC | 0,4990 | 3 | 2 | 0,1547 | 2,5 | 0,2312 | 2 |
| 8 | TC | 0,0478 | 4 | 2 | 0,2226 | 4 | 0,0004 | 1 |
| 9 | TC | 0,6889 | 3 | 2 | 0,4125 | 3 | 0,0002 | |
| 10 | TC | 1,0405 | 4 | 3 | 0,3362 | 3 | 0,0004 | |
| 11 | TC | 0,5221 | 4 | 3 | 1,1994 | 3 | 0,0001 | |
| 12 | TC | 0,8841 | 3 | 2,5 | 0,4577 | 3 | 0,0060 | |
| 13 | TC | 0,4820 | 4 | 2 | 0,3504 | 3 | 0,0806 | |
| 14 | TC | 0,5792 | 3 | 3 | 0,6985 | 3,5 | 0,0058 | |
| 15 | TC | 0,0001 | 0 | 2 | 0,0018 | 0 | 0,0150 | |
| 16 | TC | 0,1985 | 3 | 2 | 0,0913 | 4 | 0,0366 | |
| 17 | TC | 0,4923 | 4 | 3 | 0,2411 | 3 | 0,0009 | |
| 18 | TC | 0,3180 | 4 | 3 | 0,3051 | 4 | 0,0155 | |
| 19 | TC | 0,3093 | 4 | 2 | 0,0299 | 2 | 0,0303 | |
| 20 | TC | 0,3888 | 3,5 | 2 | 0,0562 | 3 | 0,0197 | |
| 21 | TC | 0,2565 | 3 | 2 | 0,0959 | 2,5 | 0,0965 | |
| 22 | TC | 0,4922 | 4 | 1,5 | 0,2530 | 3 | 0,0006 | |
| 23 | TC | 0,2226 | 2 | 3 | 0,3397 | 3,5 | 0,0036 | |
| 24 | TC | 0,4838 | 3 | 3 | 0,4153 | 4 | 0,0143 | |
| 25 | TC | 0,4922 | 4 | 2 | 0,1310 | 2,5 | 0,0010 | |
| 26 | TC | 1,2503 | 3,5 | 1,5 | 0,2180 | 3 | 0,0002 | |
| 27 | TC | 0,2866 | ? | ? | 0,3915 | 3 | 0,0238 | |
| 28 | TC | 0,5634 | 3 | 3 | 0,1992 | | 0,0040 | |
| 29 | TC | 0,3861 | 3 | 3 | 0,4888 | 4 | 0,0030 | |
| 30 | TC | 0,0000 | 0 | 0 | 0,0003 | 0 | 0,0032 | |
| 36 | AC | 0,0891 | 4 | 2 | 0,0179 | 0 | 0,0271 | |
| 37 | AC | 0,0001 | 0 | 3 | 0,0125 | 0 | 0,0233 | |
| 38 | AC | 0,0260 | 0 | 2 | 0,0124 | | 0,0564 | |
| 39 | AC | 0,2513 | | | 0,4656 | 2 | 0,0010 | |
| 41 | AC | 0,1370 | 3 | 3 | 0,2336 | 4 | 0,0017 | |
| 42 | AC | 0,0000 | 1 | 3 | 0,0082 | 0 | 0,0001 | |
| 44 | AC | 0,5595 | 3,5 | 2 | 0,5220 | 4 | 0,0125 | |
| 45 | AC | 0,0000 | 0 | 1 | 0,0068 | 1 | 0,0385 | |
| 46 | AC | 0,2647 | 3 | 2 | 0,0280 | 2 | 0,2521 | |
| 48 | AC | 0,9641 | 2,5 | 1 | 0,2947 | | 0,0083 | |
| 49 | AC | 0,0549 | 3 | 2 | 0,0019 | 0 | 0,0012 | |
| 50 | AC | 0,2135 | 4 | 3 | 0,7131 | 4 | 0,0004 | 3 |
| 51 | AC | 0,0001 | 1 | 0 | 0,0004 | 0 | 0,0695 | |
| 52 | AC | 0,0012 | 1 | 1 | 0,0129 | 0 | 0,0932 | |
| 53 | AC | 0,4153 | 3 | 2 | 0,6251 | 4 | 0,0057 | |
| 54 | AC | 0,0000 | 0 | 1 | 0,0014 | 0 | 0,5874 | 2 |
| 56 | AC | 0,5293 | 3 | 3 | 0,2150 | | 0,0256 | |
| 57 | LCNEC | 0,0000 | 0 | 0 | 0,0323 | 2 | 0,0007 | |
| 58 | LCNEC | 0,0003 | 1 | 1 | 0,0530 | 1 | 0,0098 | |
| 59 | LCNEC | 0,0000 | 1 | 1 | 0,0177 | | 0,2135 | |
| 60 | LCNEC | 0,0000 | 0 | 0 | 0,0180 | | 0,0068 | |
| 61 | LCNEC | 0,0000 | 0 | 1 | 0,0084 | | 0,0006 | |
| 63 | LCNEC | 0,0000 | 0 | 1 | 0,0255 | | 0,0003 | |
| 64 | Mixed | 0,0000 | 0 | 1 | 0,0337 | 0 | 0,0002 | |
| | LCNEC- | | | | | | | |
| | SCLC | | | | | | | |
| 65 | SCLC | 0,0000 | 0 | 1 | 0,0043 | | 0,0149 | |
| 66 | SCLC | 0,0000 | 0 | 1 | 0,0011 | | 0,0193 | |
| 68 | SCLC | 0,0000 | 0 | 1 | 0,0175 | 0 | 0,0541 | |
| 69 | SCLC | 0,0022 | 0,5 | 2 | 0,0029 | | 0,0156 | |

| | | | | | | | | |
|---|---|---|---|---|---|---|---|---|
| Abbreviations used: AC, atypical carcinoid; IHC, immunohistochemistry; LCNEC, large cell neuroendocrine carcinoma; SCLC, small cell lung cancer;TC, typical carcinoid; | | | | | | | | |

A summary of the immunohistochemistry results is shown in Table 2.

**Table 2**

| **A CD44** | **Number of cases** | **Membrane staining > 1** | **Membrane staining ≤1** | **Nuclear staining, membrane staining ≤ 1** |
|---|---|---|---|---|
| Carcinoids | 184 | 120 | 64 | 6 |
| TC | 137 | 98 | 39 | 3 |
| AC | 45 | 21 | 24 | 3 |
| Carcinomas | 33 | 3 | 30 | 1 |
| LCNEC | 14 | 3 | 11 | 0 |
| SCLC | 19 | 0 | 19 | 1 |
| **Total** | **217** | **123** | **94** | **7** |
| | | | | |
| | | | | |

| **B OTP** | **Number of cases** | **Nuclear staining > 1** | **Cytoplasmic staining > 1 and nuclear staining ≤ 1** | **Cytoplasmic and nuclear staining ≤ 1** |
|---|---|---|---|---|
| Carcinoids | 200 | 149 | 37 | 13 |
| TC | 147 | 119 | 19 | 9 |
| AC | 50 | 28 | 17 | 4 |
| Carcinomas | 52 | 1 | 7 | 44 |
| LCNEC | 20 | 0 | 3 | 17 |
| SCLC | 32 | 1 | 4 | 27 |
| **Total** | **251** | **150** | **44** | **57** |

| **C RET** | **Number of cases** | **Staining >1** | **Staining ≤1** | |
|---|---|---|---|---|
| | | | | |
| Carcinoids | 5 | 4 | 1 | |
| TC | 3 | 2 | 1 | |
| AC | 2 | 2 | 0 | |
| Carcinomas | 0 | 0 | 0 | |
| LCNEC | 0 | 0 | 0 | |
| SCLC | 0 | 0 | 0 | |
| **Total** | **5** | **4** | **1** | |

| | | | | |
|---|---|---|---|---|
| Abbreviations used: AC, atypical carcinoids; LCNEC, large cell neuroendocrine carcinomas; SCLC, small cell lung cancers; TC, typical carcinoids | | | | |

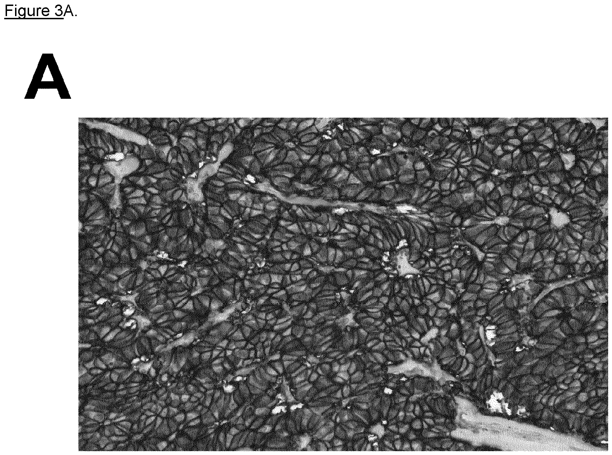

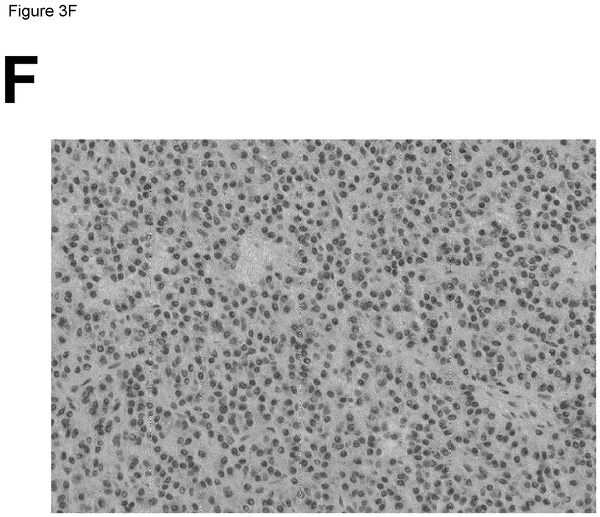

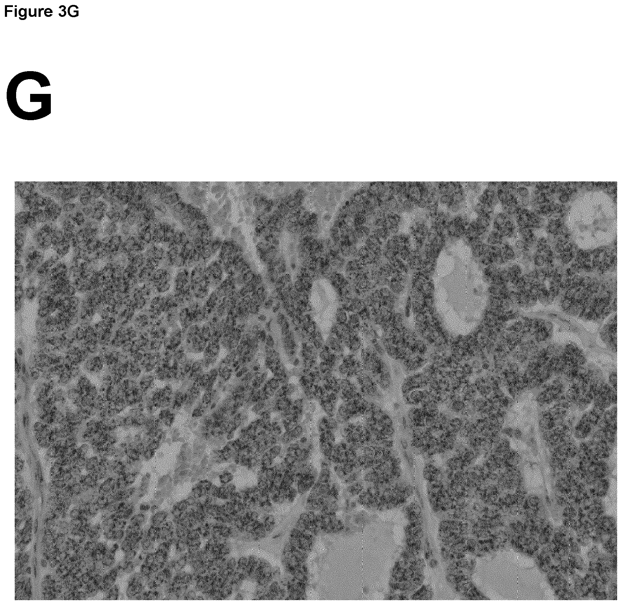

CD44 displayed a typical membrane-localized staining pattern that was very strong for most positive cases (Figure 3A). With respect to the carcinoid tumors, 64 cases (34.8%) displayed a staining intensity lower or equal to 1, while 120 cases (65.2%) were regarded CD44-positive with an intensity > 1. Within the high grade NETs these numbers were 30 (90.9%) and 3 (9.1 %), respectively (Table 2). Some cases that lost membranous expression displayed weak to moderate nuclear positivity. Due to the intense membranous staining in the majority of cases, nuclear positivity could only been assessed for membrane-negative CD44 cases. Nuclear CD44 positivity was present in < 10% of membrane-negative cases.

OTP staining was more heterogeneous and could be expressed in both the nucleus and the cytoplasm, in the nucleus with weak or absent cytoplasmic staining, dislocated from the nucleus to the cytoplasm or completely absent. With respect to the carcinoid tumors, there was nuclear OTP expression in 149 cases (74.5%), dislocation to the cytoplasm in 37 cases (18.5%) and absence of staining in 13 cases (6.5%) (Table 2). In the high grade lung NETs, these numbers were 1 (1.9%), 7 (13.5%) and 44 (84.6%), respectively. When nuclear staining was present, there was most often staining in >90% of tumor cells, although a minority of samples showed more heterogeneous staining.

RET staining was cytoplasmic and often punctuate. It has so far only been analyzed within a small number of cases (Table 2).

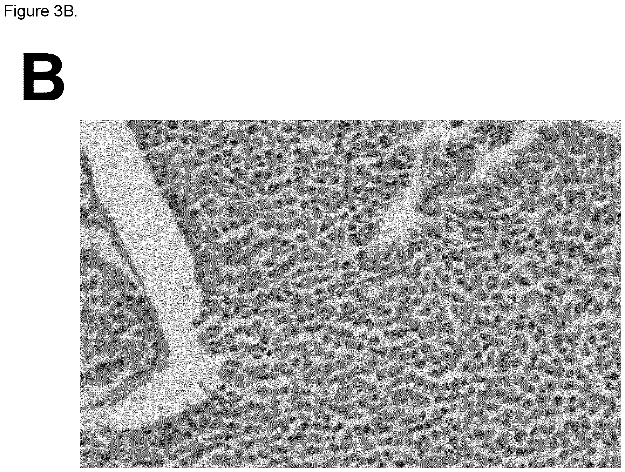

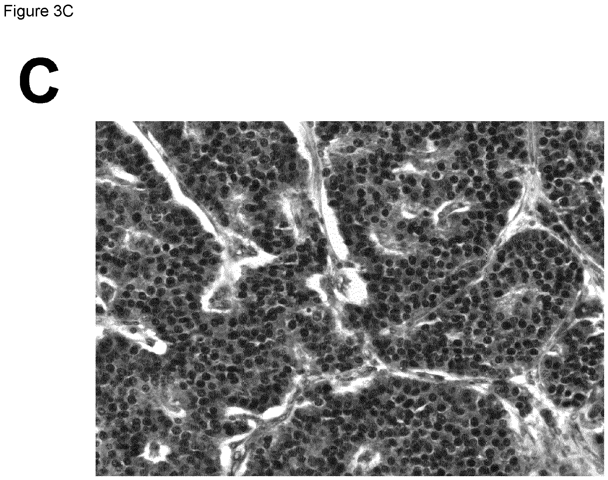

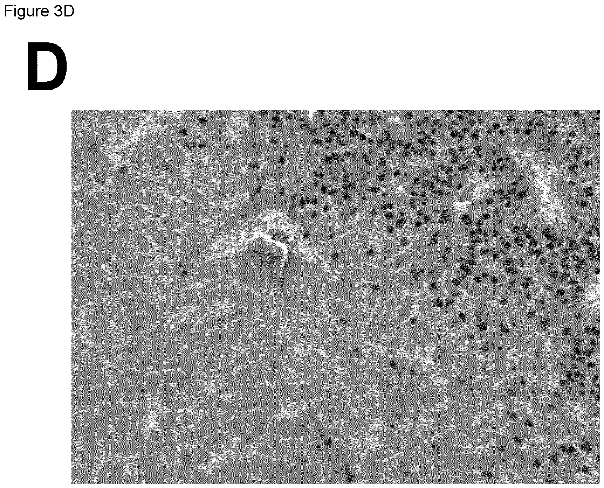

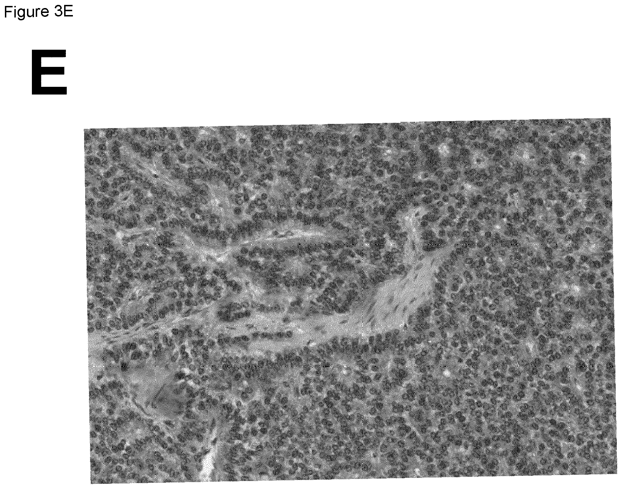

Absence of CD44 staining was correlated with low 20-year overall survival in the complete group of NETs (p<0.00001) and within the subgroup of carcinoid tumors (p=0.00023; Figure 4A). Within the carcinoid tumors, absence of CD44s was also strongly correlated with both metastasis and distant metastasis (p<0.0001). Absence of nuclear OTP staining was also correlated to poor prognosis, both within the complete group and within carcinoid tumors (both p≤0.0020; Figure 3B). Absence of cytoplasmic OTP staining, , was a bit less strongly correlated to an unfavorable disease outcome, because not all nuclear positive cases, associated with a favorable disease outcome, also displayed cytoplasmic OTP expression (p=0.0018, Figure 3C). OTP could also be prognostically subdivided in groups of tumors with nuclear staining, irrespectively of the simultaneous presence of cytoplasmic OTP, tumors with retention of cytoplasmic staining but loss of nuclear staining, and tumors with complete loss of OTP staining. This was also highly correlated with a poor patient outcome within the group of pulmonary carcinoids (p=0.0033; Figure 3D) and the total group of tumors (p<0.00001; Figure 3E). Histopathologic classification into TCs and ACs was also correlated to an adverse disease outcome (p<0.0001), but only after reclassification by experienced pathologists.

SEQUENCE LISTING

<110>  Universiteit Maastricht

       academisch ziekenhuis Maastricht

<120>  IN VITRO METHOD FOR DETERMINING DISEASE OUTCOME IN PULMONARY

       CARCINOIDS

<130>  225 EPP0

<160>  14

<170>  PatentIn version 3.5

<210>  1

<211>  17

<212>  DNA

<213>  homo sapiens

<400>  1

gcacagagcc tcgcctt                                                    17

<210>  2

<211>  17

<212>  DNA

<213>  homo sapiens

<400>  2

gttgtcgacg acgagcg                                                    17

<210>  3

<211>  22

<212>  DNA

<213>  homo sapiens

<400>  3

tttcatctgc actgccaaga ct                                              22

<210>  4

<211>  22

<212>  DNA

<213>  homo sapiens

<400>  4

ttcatgcctt ctttcacttt gc                                                22

<210>  5

<211>  20

<212>  DNA

<213>  homo sapiens

<400>  5

cgtcccacct agaatctgct                                                   20

<210>  6

<211>  20

<212>  DNA

<213>  homo sapiens

<400>  6

ttgctcacaa aggtcacagg                                                   20

<210>  7

<211>  22

<212>  DNA

<213>  homo sapiens

<400>  7

cactggcaaa acaatgcaga ct                                                22

<210>  8

<211>  25

<212>  DNA

<213>  homo sapiens

<400>  8

gtctggctta tatccaacac ttcgt 25

<210> 9

<211> 19

<212> DNA

<213> homo sapiens

<400> 9

gacaagtttt ggtggcacg 19

<210> 10

<211> 20

<212> DNA

<213> homo sapiens

<400> 10

cacgtggaat acacctgcaa 20

<210> 11

<211> 20

<212> DNA

<213> homo sapiens

<400> 11

cgacatcttt atgcgtgagg 20

<210> 12

<211> 20

<212> DNA

<213> homo sapiens

<400> 12

tttttgcgct tcttccactt 20

<210> 13

<211> 22

<212>  DNA

<213>  homo sapiens

<400>  13

tgcccagtac ctactccctc tc                                                22

<210>  14

<211>  15

<212>  DNA

<213>  homo sapiens

<400>  14

gaccacccct agcgt                                                        15

### References and literature incorporated by reference.

1. Righi, L., Volante, M., Rapa, I., Scagliotti, G. V. & Papotti, M. Neuro-endocrine tumours of the lung. A review of relevant pathological and molecular data. Virchows Arch. 451 Suppl 1, S51-S59 (2007).
2. Beasley, M. B. et al. Pathology and genetics of tumours of the lung, pleura, thymus and heart. Travis, W. D., Brambilla, E., Muller-Harmelink, H. K. & Harris, C. C. (eds.) (IARC Press, Lyon:2004).
3. Travis, W. D. Advances in neuroendocrine lung tumors. Ann. Oncol. 21 Suppl 7, vii65-vii71 (2010).
4. Granberg, D. et al. Experience in treatment of metastatic pulmonary carcinoid tumors. Ann. Oncol. 12, 1383-1391 (2001).
5. Beasley, M. B. et al. Pulmonary atypical carcinoid: predictors of survival in 106 cases. Hum. Pathol. 31, 1255-1265 (2000).
6. Lim, E. et al. Proceedings of the IASLC International Workshop on Advances in Pulmonary Neuroendocrine Tumors 2007. J. Thorac. Oncol. 3, 1194-1201 (2008).
7. Rugge, M. et al. Bronchopulmonary carcinoid: phenotype and long-term outcome in a single-institution series of Italian patients. Clin. Cancer Res. 14, 149-154 (2008).
8. Brambilla, E. et al. Apoptosis-related factors p53, Bcl2, and Bax in neuroendocrine lung tumors. Am. J. Pathol. 149, 1941-1952 (1996).
9. Granberg, D., Wilander, E., Oberg, K. & Skogseid, B. Decreased survival in patients with CD44-negative typical bronchial carcinoid tumors. Int. J. Cancer 84, 484-488 (1999).
10. Pelosi, G. et al. Alteration of the E-cadherin/beta-catenin cell adhesion system is common in pulmonary neuroendocrine tumors and is an independent predictor of lymph node metastasis in atypical carcinoids. Cancer 103, 1154-1164 (2005).
11. Debelenko, L. V. et al. Identification of MEN1 gene mutations in sporadic carcinoid tumors of the lung. Hum. Mol. Genet. 6, 2285-2290 (1997).
12. Görtz, B. et al. Mutations and allelic deletions of the MEN1 gene are associated with a subset of sporadic endocrine pancreatic and neuroendocrine tumors and not restricted to foregut neoplasms. Am. J. Pathol. 154, 429-436 (1999).
13. Johnen, G., Krismann, M., Jaworska, M. & Muller, K. M. CGH-Befunde bei neuroendokrinen Tumoren der Lunge. Pathologe 24, 303-307 (2003).
14. Petzmann, S., Ullmann, R., Halbwedl, I. & Popper, H. H. Analysis of chromosome-11 aberrations in pulmonary and gastrointestinal carcinoids: an array comparative genomic hybridization-based study. Virchows Arch. 445, 151-159 (2004).
15. Swarts, D. R. et al. Deletions of 11 q22.3-Q25 Are Associated with Atypical Lung Carcinoids and Poor Clinical Outcome. Am. J. Pathol. (2011).
16. Walch, A. K. et al. Typical and atypical carcinoid tumors of the lung are characterized by 11 q deletions as detected by comparative genomic hybridization. Am. J. Pathol. 153, 1089-1098 (1998).
17. Zhao, J. et al. Genomic alterations in well-differentiated gastrointestinal and bronchial neuroendocrine tumors (carcinoids): marked differences indicating diversity in molecular pathogenesis. Am. J. Pathol. 157, 1431-1438 (2000).
18. Sachithanandan, N., Harle, R. A. & Burgess, J. R. Bronchopulmonary carcinoid in multiple endocrine neoplasia type 1. Cancer 103, 509-515 (2005).
19. Swarts, D. R. et al. Deletions of 11 q22.3-Q25 Are Associated with Atypical Lung Carcinoids and Poor Clinical Outcome. Am. J. Pathol. (2011).
20. Lopez, J. R. et al. Spectral karyotypic and comparative genomic analysis of the endocrine pancreatic tumor cell line BON-1. Neuroendocrinology 91, 131-141 (2010).
21. van Koetsveld, P. M. et al. Potent inhibitory effects of type I interferons on human adrenocortical carcinoma cell growth. J. Clin. Endocrinol. Metab 91, 4537-4543 (2006).
22. Vandesompele, J. et al. Accurate normalization of real-time quantitative RT-PCR data by geometric averaging of multiple internal control genes. Genome Biol. 3, RESEARCH0034 (2002).
23. Granberg, D., Wilander, E., Oberg, K. & Skogseid, B. Prognostic markers in patients with typical bronchial carcinoid tumors. J. Clin. Endocrinol. Metab 85, 3425-3430 (2000).
24. Swarts, D. R. A. et al. Deletions of 11 q22.3-q25 Are Associated with Atypical Lung Carcinoids and Poor Clinical Outcome. Am. J. Pathol. 179, 1129-1137 (2011).
25. Ponta, H., Sherman, L. & Herrlich, P. A. CD44: from adhesion molecules to signalling regulators. Nat. Rev. Mol. Cell Biol. 4, 33-45 (2003).
26. Zoller, M. CD44: can a cancer-initiating cell profit from an abundantly expressed molecule? Nat. Rev. Cancer 11, 254-267 (2011).
27. Brown, R. L. et al. CD44 splice isoform switching in human and mouse epithelium is essential for epithelial-mesenchymal transition and breast cancer progression. J. Clin. Invest 121, 1064-1074 (2011).
28. Schmits, R. et al. CD44 regulates hematopoietic progenitor distribution, granuloma formation, and tumorigenicity. Blood 90, 2217-2233 (1997).
29. Sun, X., Gong, Y., Talamonti, M. S. & Rao, M. S. Expression of cell adhesion molecules, CD44s and E-cadherin, and microvessel density in carcinoid tumors. Mod. Pathol. 15, 1333-1338 (2002).
30. Fasano, M. et al. CD44 and its v6 spliced variant in lung tumors: a role in histogenesis? Cancer 80, 34-41 (1997).
31. Nguyen, V. N., Mirejovsky, T., Melinova, L. & Mandys, V. CD44 and its v6 spliced variant in lung carcinomas: relation to NCAM, CEA, EMA and UP1 and prognostic significance. Neoplasma 47, 400-408 (2000).
32. Okamoto, I. et al. Proteolytic release of CD44 intracellular domain and its role in the CD44 signaling pathway. J. Cell Biol. 155, 755-762 (2001).
33. Lin, X. et al. Identification, chromosomal assignment, and expression analysis of the human homeodomain-containing gene Orthopedia (OTP). Genomics 60, 96-104 (1999).
34. Acampora, D. et al. Progressive impairment of developing neuroendocrine cell lineages in the hypothalamus of mice lacking the Orthopedia gene. Genes Dev. 13, 2787-2800 (1999).
35. Wang, W. & Lufkin, T. The murine Otp homeobox gene plays an essential role in the specification of neuronal cell lineages in the developing hypothalamus. Dev. Biol. 227, 432-449 (2000).
36. Blechman, J. et al. Specification of hypothalamic neurons by dual regulation of the homeodomain protein Orthopedia. Development 134, 4417-4426 (2007).
37. Amir-Zilberstein, L. et al. Homeodomain protein otp and activity-dependent splicing modulate neuronal adaptation to stress. Neuron 73, 279-291 (2012).
38. Arighi, E., Borrello, M. G. & Sariola, H. RET tyrosine kinase signaling in development and cancer. Cytokine Growth Factor Rev. 16, 441-467 (2005).
39. Marx, S. J. Molecular genetics of multiple endocrine neoplasia types 1 and 2. Nat. Rev. Cancer 5, 367-375 (2005).
40. Lemos, M. C. & Thakker, R. V. Multiple endocrine neoplasia type 1 (MEN1): analysis of 1336 mutations reported in the first decade following identification of the gene. Hum. Mutat. 29, 22-32 (2008).
41. Ichihara, M., Murakumo, Y. & Takahashi, M. RET and neuroendocrine tumors. Cancer Lett. 204, 197-211 (2004).
42. Komminoth, P. et al. RET proto-oncogene point mutations in sporadic neuroendocrine tumors. J. Clin. Endocrinol. Metab 81, 2041-2046 (1996).
43. Futami, H., Egawa, S. & Yamaguchi, K. A Novel Point Mutation of the Ret Protooncogene in Small-Cell Lung-Carcinoma Cell-Lines. Proceedings of the Japan Academy Series B-Physical and Biological Sciences 70, 210-214 (1994).
44. Futami, H. et al. A novel somatic point mutation of the RET Proto-oncogene in tumor tissues of small cell lung cancer patients. Jpn. J. Cancer Res. 86, 1127-1130 (1995).
45. Futami, H. et al. Allelic loss of DNA locus of the RET proto-oncogene in small cell lung cancer. Cancer Lett. 195, 59-65 (2003).
46. Mulligan, L. M. et al. Investigation of the genes for RET and its ligand complex, GDNF/GFR alpha-I, in small cell lung carcinoma. Genes Chromosomes Cancer 21, 326-332 (1998).
47. Cao, Y. et al. Nuclear-cytoplasmic shuttling of menin regulates nuclear translocation of {beta}-catenin. Mol. Cell Biol. 29, 5477-5487 (2009).
48. Gujral, T. S. et al. A novel RET kinase-beta-catenin signaling pathway contributes to tumorigenesis in thyroid carcinoma. Cancer Res. 68, 1338-1346 (2008).
49. Salon, C. et al. E-cadherin-beta-catenin adhesion complex in neuroendocrine tumors of the lung: a suggested role upon local invasion and metastasis. Hum. Pathol. 35, 1148-1155 (2004).

SEQUENCE LISTING

<110>  Universiteit Maastricht
       Academisch Ziekenhuis Maastricht

<120>  IN VITRO METHOD FOR DETERMINING DISEASE OUTCOME IN PULMONARY
       CARCINOIDS

<130>  225 EPPO

<160>  14

<170>  PatentIn version 3.5

<210>  1
<211>  17
<212>  DNA
<213>  homo sapiens

<400>  1
gcacagagcc tcgcctt                                                    17


<210>  2
<211>  17
<212>  DNA
<213>  homo sapiens

<400>  2
gttgtcgacg acgagcg                                                    17


<210>  3
<211>  22
<212>  DNA
<213>  homo sapiens

<400>  3
tttcatctgc actgccaaga ct                                              22


<210>  4
<211>  22
<212>  DNA
<213>  homo sapiens

<400>  4
ttcatgcctt ctttcacttt gc                                              22


<210>  5
<211>  20
<212>  DNA
<213>  homo sapiens

<400>  5
cgtcccacct agaatctgct                                                 20


<210>  6
<211>  20
<212>  DNA
<213>  homo sapiens

<400>  6
ttgctcacaa aggtcacagg                                                 20


<210>  7

<211>  22
<212>  DNA
<213>  homo sapiens

<400>  7
cactggcaaa acaatgcaga ct                                                22


<210>  8
<211>  25
<212>  DNA
<213>  homo sapiens

<400>  8
gtctggctta tatccaacac ttcgt                                             25


<210>  9
<211>  19
<212>  DNA
<213>  homo sapiens

<400>  9
gacaagtttt ggtggcacg                                                    19


<210>  10
<211>  20
<212>  DNA
<213>  homo sapiens

<400>  10
cacgtggaat acacctgcaa                                                   20


<210>  11
<211>  20
<212>  DNA
<213>  homo sapiens

<400>  11
cgacatcttt atgcgtgagg                                                   20


<210>  12
<211>  20
<212>  DNA
<213>  homo sapiens

<400>  12
tttttgcgct tcttccactt                                                   20


<210>  13
<211>  22
<212>  DNA
<213>  homo sapiens

<400>  13
tgcccagtac ctactccctc tc                                                22


<210>  14
<211>  15
<212>  DNA
<213>  homo sapiens

<400>  14
gaccacccct agcgt                                                        15

## Claims

1. In vitro method for predicting the outcome of a disease by determining the level of a marker in a sample wherein the disease is a pulmonary carcinoid tumor and wherein the marker is an RNA or protein expression product of a RET proto-oncogene or an RNA or protein expression product of an Orthopedia homeobox (OTP) gene.

2. Method according to claim 1 wherein the level of the marker is above a certain reference value for the RNA or protein expression product of the RET proto-oncogene and/or wherein the level of the marker is below a certain reference value for RNA or protein expression product of the Orthopedia homeobox (OTP) gene.

3. Method according to claim 2 wherein the reference value is the mean level of expression of the RNA or protein expression product of the corresponding gene in at least one normal individual.
